## Europäisches Patentamt
## European Patent Office
## Office européen des brevets

(19)

(11) Publication number: **0 000 439**
**B1**

(12)

# EUROPEAN PATENT SPECIFICATION

(45) Date of publication of patent specification: **02.09.81**

(21) Application number: **78300127.4**

(22) Date of filing: **07.07.78**

(51) Int. Cl.³: **C 07 C 103/52,**
**A 61 K 37/26**

(54) Method of purifying an insulin-containing aqueous ethanolic raw extract from pancreas glands.

(30) Priority: **08.07.77 DK 3114/77**

(43) Date of publication of application:
**24.01.79 Bulletin 79/2**

(45) Publication of the grant of the European patent:
**02.09.81 Bulletin 81/35**

(84) Designated Contracting States:
**BE CH DE FR GB LU NL SE**

(56) References cited:
**BE - A - 850 387**
**DE - A - 2 212 695**
**US - A - 3 876 623**

(73) Proprietor: **LABORATORIOS LEO S.A.**
**Avda. de Pio XII No.99**
**Madrid (ES)**

(72) Inventor: **Jensen, Willy Henry**
**c/o LABORATORIOS LEO S.A. APARTADO 439**
**Avda. de Pio XII No.99 Madrid (ES)**

(74) Representative: **Harrison, David Christopher et al,**
**MEWBURN ELLIS & CO 70 & 72 Chancery Lane**
**London WC2A 1AD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Method of purifying an insulin-containing aqueous ethanolic raw extract from pancreas glands

The invention concerns a method of purifying an insulin-containing aqueous ethanolic raw extract from pancreas glands, which method is useful as a purification step in the working up of the extract to pure monocomponent insulin.

The term "raw insulin extract" is used herein in the usual meaning, i.e. the extract obtained by treating pancreas glands with an aqueous organic solvent, especially aqueous ethanol, usually in a concentration of 60 to 80%, and said term comprises also extracts which, besides being freed from fat, have been subjected to various treatments, for example precipitation of proteins different from insulin by a change of pH value, the so-called pH—8 precipitation, and possibly reverse osmosis, in such a manner, however, that the insulin during these treatments has remained in liquid phase. Thus, the term does not comprise insulin-containing solutions formed by dissolution in a solvent of purified solid insulin isolated from the original extract.

It is now generally assumed that the antigenicity in insulin mostly due to impurities in the preparations, whereas it was formerly believed that the insulin anti-bodies were produced by the insulin as such. These impurities may be accompanying proteins from pancreas distinct from insulin, proinsulin which is a precursor of insulin, intermediate insulin, the dimer, arginine insulin, ethylester insulin, desamido insulin desamidised to various extents, other insulin modifications and coloured substances.

Efforts have therefore been made in order to produce insulin preparations consisting of pure insulin, the so-called monocomponent insulin, free of impurities and accompanying substances of any kind.

For that purpose it has been proposed to subject amorphous or crystalline insulin prepared in a conventional manner to an extensive further purification, e.g. by ion exchange treatment, gel filtration using a so-called molecular sieve or partition-chromatography (German: Verteilungschromatographie).

Purification by partition-chromatography is, for instance, described in German Offenlegungsschrift 2 212 695. It is known therefrom to subject a solution formed by dissolving solid, still impure insulin in an aqueous solvent system which besides alcohols with 4—5 carbon atoms contains carboxylic acids with 1—3 carbon atoms and/or ammonia or an organic base with a $pK_a$-value between about 5.1 and 11, to partition-chromatography using as the gel Sephadex® LH—20. The said gel is dextran cross-linked with epichlorohydrin and containing hydroxypropyl groups attached by ether linkages to the glucose units of the dextran chains.

The highly purified insulins resulting from the abovementioned known purification methods show a strong decrease in antigenicity but not a complete removal thereof. This is no doubt due to the fact that in spite of the purification steps, the purified preparations still contain substances different from insulin.

The applicants have previously developed a method of preparing very pure monocomponent insulin, cf. Danish patent application No.141/77 published 16 July 1977, German Offenlegungsschrift No.27 01 092 published 28 July 1977 and Belgian patent specification No.850 387 published 2 May 1977.

The said method is based on the recognition that some of the impurities present in insulin are, when using the conventional methods, formed during the recovery itself of insulin, for which reason it is essential to carry out the preparation of insulin in such a manner that the insulin, form the extraction from the pancreas glands until obtention of the final product, is only subjected to conditions of such a nature that they do not cause the formation of decomposition products, aggregates etc.

The method is characterized in that the insulin-containing extract prepared from the pancreas glands is worked up to pure insulin in such a way that the insulin is maintained in a solvent environment during the whole processing, until the final recovery of the insulin, the undesired substances being from the beginning of the process until the end removed from the different solvents used.

In the said process it is not a question of a purification of the insulin itself, but a purification of the obtained insulin extract takes place to remove dissolved impurities and fat and undesirable proteins and derivatives of insulin, until the pure insulin remains alone in the extract (or in another liquid phase).

The process can for instance, be carried out by subjecting the extract to a treatment for removal of fat consisting in cooling of the extract to a low temperature, for example between −25°C and −45°C, followed by separation of the crystallized fat; concentrating the extract by means of reverse osmosis, the insulin being simultaneously separated from impurities present in the extract, substances having a larger molecule than insulin as well as substances having a smaller molecule than insulin; washing the insulin-containing concentrate from the reverse osmosis in a reverse osmosis plant for partial removal of coloured substances and salts form the concentrate; subjecting the purified concentrate to further purification by means of ion exchange under conditions at which the insulin remains in liquid phase; and finally recovering the insulin from the concentrated purified solution by precipitation with metal ions, preferably zinc ions, under strong cooling to e.g. −30°C to −45°C.

In an effective separation on ion exchanger of the insulin in such a raw insulin extract from impurities it is, however, difficult to avoid a loss of insulin, since in the case of an extended period of separation aggregates between the insulin molecules may be formed, which aggregates can only with difficulty be eluted later on.

The applicants have therefore set themselves the task of finding a purification method which can supplement or partly replace a treatment of the raw extract with ion exchangers, so that an effective separation is obtained without a loss of insulin or with less loss of insulin than before.

It has been found that a purification of an insulin-containing ethanolic raw extract with approximately a 100% yield of insulin can be obtained by subjecting the extract to gel filtration using a particular gel and observing particular conditions.

The method according to the invention is characterized in that the insulin-containing ethanolic raw extract after having been cooled down to remove fat by crystallization followed by separation of the fat crystals from the extract and optionally having been further pre-purified is subjected at an ethanol concentration being between 60 and 80 per cent by volume, to gel filtration on a gel swollen in ethanol or in aqueous ethanol, preferably with an ethanol concentration of at least 60 per cent by volume, and preferably but not necessarily having the same concentration and the same pH—value as the extractant used, use being made as an eluant likewise of ethanol or of aqueous ethanol, preferably with an ethanol concentration of at least 60 per cent by volume.

The gel used is a bead-formed gel possessing both hydrophilic and lipophilic properties. It is a dextran cross-linked with epichlorohydrin and containing hydroxypropyl groups attached to the glucose units of the dextran chains by ether linkages having an exclusion limit of about 10,000 MW and a water regain value of 5.3 to 6.3 ml/g. Such a gel is Sephadex® LH—60, produced by Pharmacia Fine Chemicals, Uppsala, Sweden.

In the gel filtration according to the invention, the insulin molecules penetrate the gel particles, being below the exclusion limit of the gel. When eluting, an insulin fraction is obtained which is totally freed from coloured substances and which contains no or practically no proteins having a larger molecule than insulin or having a smaller molecule than insulin, but only insulin derivatives with practically the same molecular size as the insulin. The yield of insulin is practically 100%.

The present method can with special advantage be used in connection with the method described in Danish patent application No. 141/77, the gel filtration supplementing or partly replacing the treatment with ion exchangers described in the specification of said application.

The gel filtration according to the present invention is, however, not restricted to the use in connection with said older method but it can be used on any aqueous ethanolic raw extract freed from fat and can constitute either the final purification step before the isolation of solid insulin or an intermediate purification step.

As regards the previously mentioned method known from German Offenlegungsschrift 2 212 695, which consists in subjecting a solution formed by dissolving solid insulin in an aqueous solvent system which, besides alcohols with 4—5 carbon atoms, contains a carboxylic acid and/or ammonia or an organic base, to partition-chromatography using Sephadex® LH—20, it is observed that that uses a separation technique different from gel filtration, being one primarily based on partition effects. The method is not suitable for purifying an insulin-containing raw-extract, inter alia because a solvent system must be used which is different from the solvents commonly used for extracting the pancreas glands. It will also be noted that Sephadex® LH—20 differs from Sephadex® LH—60 by having a lower exclusion limit than the latter, cf. the Offenlegungsschrift page 4, third complete paragraph, so that the insulin molecules will not penetrate the gel.

In the purification according to the invention of a raw insulin extract the procedure can e.g. be as follows:

The gel is brought to swell in ethanol or in aqueous ethanol preferably with an ethanol concentration of at least 60 per cent by volume, for example a mixture corresponding to that used for the extraction of the pancreas glands or possibly a mixture with a slightly higher concentration of ethanol.

The column is prepared in the usual way, the extract — with an ethanol concentration between 60 and 80 per cent by volume — is supplied and the column is filled with the eluant, viz. ethanol or aqueous ethanol. The eluant may be the same as the swelling agent.

The pH-value of the liquid phase in the Sephadex bed is preferably between 3 and 8. (Eluates having a pH-value below about 3 are too acid for immediate application to ion exchangers, and at pH-values about 8 the insulin will be partly destroyed).

The insulin is eluted and the fraction collected. It is completely free of coloured substances. An almost 100% yield of insulin is obtained.

For obtaining pure monocomponent insulin the eluate is further purified, e.g. by means of ion exchanger, and the insulin is finally isolated, for example by precipitation.

## Example

An insulin-containing raw extract obtained by treating frozen, finely comminuted pancreas glands from hogs with 85% ethanol acidified to a pH-value of about 3 with $H_2SO_4$ (2 l 85% ethanol/1 kg glands; giving with the water in the glands an aqueous 65% ethanol extract), removing fat from the extract by cooling to −30°C followed by separation of the fat crystals formed and concentrating to 1/10 of the origcal volume by reverse osmosis, was subjected to gel filtration as follows:

50 l of the above concentrate was applied to a column (diameter 45 cm, height 75 cm) of Sephadex® LH—60 swelled in and equilibrated with 65% ethanol (acetate buffer, ion strength 0,025, pH-value 7,3).

Elution was carried out with an eluant, which was the same as the swelling agent.

The fractions containing the insulin peak were collected. They were totally free of coloured substances and contained no or practically no proteins having a larger molecule than insulin or having a smaller molecule than insulin.

**Claims**

1. process of purifying an insulin-containing ethanolic raw extract from pancreas glands, characterized in that the extract after having been cooled down to removed fat by crystallization followed by separation of the fat crystals from the extract and optionally having been further pre-purified is subjected at an ethanol concentration between 60 and 80 per cent by volume to gel filtration on a gel swollen in ethanol or in aqueous ethanol, the said gel being dextran cross-linked with epichlorohydrin and containing hydroxypropyl groups attached to the glucose units of the dextran chain by ether linkages, having an exclusion limit of about 10,000 MW and a water regain value of 5.3 to 6.3 ml/g, the eluant used being ethanol or aqueous ethanol.

2. A process according to claim 1 wherein the gel was swollen in aqueous ethanol with a concentration of at least 60 per cent by volume.

3. A process according to claim 1 or claim 2 wherein the gel was swollen in aqueous ethanol having the same cocentration and pH value as the extractant.

4. A process according to any one of the preceding claims wherein the eluant used is aqueous ethanol with an ethanol concentration of at least 60 per cent by volume.

**Revendications**

1. Procéde pour la purification d'un extrait étanolique brut de glandes pancréatiques, contenant de l'insuline, caractérisé en ce que l'extrait, après avoir été réfrigéré pour éliminer les graisses par cristallisation suivie de séparation des cristaux de graisse à partir de l'extrait, et éventuellement après avoir été en outre pré-purifié, est, avec une concentration d'éthanol comprise entre 60 et 80 pour cent en volume, soumis à une filtration au gel sur un gel gonflé dans de l'éthanol ou dans de l'éthanol aqueux, ledit gel étant du dextrane transversalement réticulé avec de l'épichlorhydrine et contenant des radicaux hydroxypropyle attachés aux motifs unitaires glucose de la chaîne de dextrane par des maillons éther, possédant une limit d'exclusion correspondant à un poids moléculaire d'environ 10 000 et une valeur de récupération d'eau de 5,3 à 6,3 ml/g, l'éluant utilisé étant de l'éthanol ou de l'thanol aqueux.

2. Procédé selon la revendication 1, caractérisé en ce que le gel est gonflé dans un éthanol aqueux avec une concentration d'au moins 60 pour cent en volume.

3. Procédé selon la revendication 1 ou 2, caractérisé en ce que le gel est gonflé dans un éthanol aqueux ayant la même concentration et la même valeur de pH que l'agent d'extraction.

4. Procéde selon l'une quelconque des revendications précédentes, caractérisé en ce que l'éluant utilisé est un éthanol aqueux ayant une concentration d'éthanol égale à au moins 60 pour cent en volume.

**Patentansprüche**

1. Verfahren zum Reinigen eines Insulin enthaltenden, wässrigethanolischen Rohextraktes von Bauchspeicheldrüsen, dadurch gekennzeichnet, dass der Extrakt nach Abkühlung zwecks Beseitigung von Fett durch Herauskristallisieren und nachfolgende Trennung der Frettkristalle vom Extrakt, und nachdem er eventuell weiter vorgereinigt worden ist, mit einer Ethanolkonzentration von 60 bis 80 Vol-%, einer Gelfiltration an einem Gel unterworfen wird, welches in Ethanol oder wässrigem Ethanol gequollen ist, welches Gel mit Epichlorhydrin vernetztes Dextran, das an den Glucoseeinheiten des Dextrans mittels Ätherbindungen geknüpfte Hydroxypropylgruppen enthält, ist, und das eine Ausschliessungsgrenze von etwa 10.000 MW und eine "water regain value" von 5,3 bis 6,3 ml/g hat, wobei das benutzte Eluiermittel Ethanol oder wässriges Ethanol ist.

2. Verfahren nach Anspruch 1, worin das Gel in wässrigem Ethanol mit einer Konzentration von mindestens 60 Vol.-% gequollen ist.

3. Verfahren nach Anspruch 1 oder 2 worin das Gel in wässrigem Ethanol mit derselben Konzentration und demselben pH-Wert wie die Extraktionsmittel gequollen ist.

4. Verfahren nach einem der vorhergehenden Ansprüche, worin das benutzte Eluiermittel wässriges Ethanol mit einer Ethanolkonzentration von mindestens 60 Vol.-% ist.